# FASCICULE DE BREVET EUROPEEN

(11) **EP 1 851 321 B1**
(45) Date de publication et mention de la délivrance du brevet: **09.09.2009**
(21) Numéro de dépôt: 06709536.4
(22) Date de dépôt: 24.02.2006
(51) Int. Cl.: C12N 15/88, C07K 14/16

(54) **EPITOPES DE VIH ET COMPOSITION PHARMACEUTIQUE LES CONTENANT**
HIV-EPITOPE UND DIESE ENTHALTENDE PHARMAZEUTISCHE ZUSAMMENSETZUNG
HIV EPITOPES AND PHARMACEUTICAL COMPOSITION CONTAINING SAME

(30) Priorité: 25.02.2005 FR 0550520
(43) Date de publication de la demande: 07.11.2007
(73) Titulaire: CENTRE NATIONAL DE LA RECHERCHE SCIENTIFIQUE (CNRS), 75794 Paris Cedex 16 (FR)
(72) Inventeur: LONE, Yu-chun, F-75017 Paris (FR); PAJOT, Anthony, F-75015 Paris (FR)
(74) Mandataire: Paris, Fabienne
(86) Numéro de dépôt international: PCT/FR2006/050167
(87) Numéro de publication internationale: WO 2006/090090

(56) Documents cités:
- WO-A-90/13564
- WO-A-20/04002415
- WO-A-20/05004592
- US-B1- 6 593 079
- STONE D.J. ET AL: "HLA-restricted epitope identification and detection of functional Tcell responses by using MHC-peptide and costimulatory microarrays" P.N.A.S., vol. 102, no. 10, 8 mars 2005 (2005-03-08), pages 3744-3749, XP002383140
- CUNLIFFE S.L. ET AL: "Optimization of peptide linker length in the production of MHC class II/peptide tetrameric complexes increases yield and stability and allows identifcation of antigen-specific CD4+ T cells in peripheral blood mononuclear cells" EUR. J. IMMUNOL., vol. 32, 2002, pages 3366-3375, XP002383141
- 7th Conference on retroviriuses and Opportunistic Infections 2000 Abstract no. 190 Kelleher A. et al. "HLA DR-1 tetramers Delineate HIV-1 specific CD3+ CD4+ cells" XP002383309
- ZAVALA-RUIZ Z. ET AL: "A hairpin turn in a class II MHC-bound peptide orients residues outside the binding groove for T cell recognition" P.N.A.S., vol. 101, no. 36, 7 septembre 2004 (2004-09-07), pages 13279-13284, XP002383142
- NOVAK E.J. ET AL: "MHC class II tetramers identify peptide specific human CD4+ T cells proliferating in response to influenza antigens" THE JOURNAL OF CLINICAL INVESTIGATION, vol. 104, 1999, pages R63-R67, XP002383143
- KELLEHER A. D. ET AL: "CLUSTURED MUTATION IN HIV-1 GAG ARE CONSISTENTY REQUIRED FOR ESCAPE FRON HLA-B27-RESTRICTED CYTOTOXIC T LYMPHOCYTE RESPONSES" J. EXP. MED., vol. 193, no. 3, 5 février 2001 (2001-02-05), pages 375-385, XP002383144
- NISHINO Y ET AL: "IN VIVO INDUCTION OF HUMAN IMMUNODEFICIENCY VIRUS TYPE 1-SPECIFIC CYTOTOXIC T LYMPHOCYTES AND DELAYED-TYPE HYPERSENSITIVITY BY A 23-AMINO ACID PEPTIDE FROM THE HIGHLY CONSERVED REGION IN MAJOR CORE PROTEIN P24" VACCINE, BUTTERWORTH SCIENTIFIC. GUILDFORD, GB, vol. 12, no. 6, mai 1994 (1994-05), pages 485-491, XP000882395 ISSN: 0264-410X

## Description

La présente invention concerne des épitopes dérivés d'antigènes viraux capables de se lier spécifiquement aux molécules HLA-DR1 et d'induire une réponse immunitaire chez des sujets de phénotypes HLA-DR1, ainsi que leurs analogues fonctionnels.

La présente invention se rapporte également à des compositions pharmaceutiques contenant des peptides comprenant ces épitopes et/ou leurs analogues fonctionnels, ainsi qu'à des méthodes de diagnostic pour déterminer l'état immunitaire d'un individu susceptible de présenter une infection virale comprenant l'utilisation de ces peptides.

Plus précisément, la présente invention se rapporte à des épitopes immunogènes dérivés d'un antigène du virus de l'immunodéficience humaine (VIH), la protéine p24, consistant en une séquence d'acides aminés choisie parmi SEQ ID NO 1, SEQ ID NO 2, SEQ ID NO 3.

Les virus du type VIH sont des virus provoquant une infection chronique, c'est-à-dire n'induisant pas une réponse immunitaire suffisante pour éliminer l'infection.

Cette infection peut aboutir à l'apparition d'un syndrome d'immunodéficience acquise (SIDA). Cette maladie est caractérisée, notamment, par une susceptibilité accrue aux infections par des agents pathogènes opportunistes, ou par l'apparition de formes agressives du sarcome de Kaposi, ou par l'apparition de lymphomes à cellule B, associée à une diminution importante du nombre de cellules T CD4+.

De nombreuses stratégies vaccinales ont été développées afin de lutter contre cette infection. La plupart de ces stratégies font appel à des moyens visant à améliorer la production d'anticorps afin de prévenir l'infection en cas d'exposition au VIH, ou à des moyens visant à activer des lymphocytes T cytotoxiques (ou CD8+ ou CTL) afin de détecter et détruire les cellules infectées, et ainsi contrôler et éliminer l'infection.

Différentes protéines de la capside virale du VIH ont été utilisées afin de produire des anticorps. Ainsi, de US 6 593 079, il est connu d'utiliser des peptides de l'antigène p24 de la protéine Gag du VIH afin de produire des anticorps.

Il apparaît que la réponse dépendante des cellules T CD4+ ou lymphocytes T-auxiliaires (ou HTL) intervient de manière importante dans le maintien des fonctions cellulaires T CD8+ adéquates et dans le contrôle de la virémie.

Cependant, les cellules T CD4+ répondant spécifiquement au VIH apparaissent être préférentiellement infectées et éliminées par le VIH, limitant ainsi la capacité des vaccins potentiels à activer les cellules T auxiliaires après le début de l'infection (Douek et al,. Nature, 2002, 417 : 95-98). Les patients chroniquement infectées sont dans l'incapacité de déclencher une réponse cellulaire de type T aussi bien contre des antigènes du VIH que contre des antigènes d'agents pathogènes opportunistes. Ainsi, la perte de la réponse HTL joue un rôle central lors du développement d'une infection à VIH.

En dépit du rôle central de ces cellules dans le contrôle de l'infection au VIH, seul un faible nombre d'épitopes restreints CD4+ immunogéniques ont été identifiés jusqu'à présent (Wilson et al., J. Virol, 2001, 75:4195-207 ; Kaufmann et al., J. Virol., 2004, 78:4463-77).

Ainsi, il existe un besoin pour de nouveaux épitopes restreints CD4+ permettant d'activer une réponse dépendante des lymphocytes T CD4+ dirigée contre des antigènes du VIH.

Il existe encore un besoin de disposer de compositions pharmaceutiques, notamment de vaccins, destinées à prévenir et/ou traiter des infections au VIH.

Par ailleurs, il est important de pouvoir qualifier la réponse immunitaire et/ou l'état immunitaire d'un patient susceptible d'être atteint par une infection à VIH afin de pouvoir déterminer sa sensibilité à la maladie et ajuster au mieux le traitement à administrer.

Ainsi, il existe un besoin de disposer d'un marqueur permettant de caractériser l'état immunitaire d'un patient.

Il existe également un besoin de disposer d'une méthode de diagnostic permettant de qualifier l'état immunitaire d'un sujet susceptible d'être atteint par une infection à VIH, ainsi que, le cas échéant, d'évaluer la réponse de son système immunitaire à un traitement.

La présente invention a notamment pour objet de satisfaire à ces besoins.

Les inventeurs ont identifié trois épitopes dérivés de l'antigène p24, issus de la protéine Gag du VIH, spécifiques du phénotype HLA-DR1.

Selon un de ses premiers aspects, la présente invention concerne un peptide comprenant au moins un épitope consistant en une séquence d'acides aminés choisie parmi SEQ ID NO 1, SEQ ID NO 2, SEQ ID NO 3, et des analogues fonctionnels de celle-ci.

Les inventeurs ont observé que ces épitopes étaient capables d'induire la prolifération, *in vitro,* de cellules T CD4+ issues de souris transgéniques HLA-DR1 invalidées H-2 classe II (*IA*β*^{b°}*) (Pajot et al, Int. Immunol, 2004, 16 : 1275-1282) vaccinées à l'aide de la protéine p24. Par ailleurs, ces peptides sont capables d'induire une réponse cellulaire de type T CD4+ à partir de cellules provenant de patients affectés par le VIH, notamment par le VIH-1, ou d'individus séronégatifs, suite à l'activation de ces cellules par des cellules présentatrices d'antigène. Les cellules T CD4+ proliférantes en réponse aux peptides selon l'invention sont caractérisées par un phénotype HLA-DR1 et la réponse biologique est caractérisée par la sécrétion d'une cytokine, l'IFN-γγ mesurée par ELISPOT.

Au sens de la présente invention, on entend désigner par "analogues fonctionnels" tout peptide ou analogue peptidique capable de mimer l'activité immunogénique des peptides selon l'invention, à savoir, notamment, de présenter la capacité de se lier aux molécules HLA-DR1, et d'induire une réponse biologique chez des lymphocytes T CD4+.

HLA signifie "Human Leukocyte Antigen" et est équivalent à CMH, "Complexe Majeur d'Histocompatibilité". Les HLA (ou CMH) correspondent à un ensemble de gènes divisés en classes I et II, présentant un très haut polymorphisme, et codant pour des molécules dont la fonction est de présenter des fragments peptidiques à la surface des cellules.

Au sens de la présente invention, on entend désigner par "immunogène", toute substance susceptible de provoquer une réponse immunitaire. Un immunogène, ou substance immunogénique, est fonctionnellement distinct d'un antigène. Un antigène est défini comme étant toute substance susceptible de se lier à un anticorps spécifique. Par conséquent, tous les antigènes sont considérés comme étant susceptibles d'induire une réponse de type anticorps, mais certains doivent être liés à un immunogène afin de pouvoir agir ainsi. Ainsi, bien que tous les immunogènes soient des antigènes, tous les antigènes ne sont pas immunogéniques.

Selon un autre de ses aspects, la présente invention se rapporte à un peptide comprenant un épitope consistant en une séquence d'acides aminés choisie parmi SEQ ID NO 1, SEQ ID NO 2, SEQ ID NO 3, et des analogues fonctionnels de celle-ci, et présentant une longueur inférieure ou égale à 24 acides aminés.

Selon un autre de ses aspects, la présente invention se rapporte à un peptide multiépitope comprenant au moins un épitope consistant en une séquence d'acides aminés choisie parmi SEQ ID NO 1, SEQ ID NO 2, SEQ ID NO 3, et des analogues fonctionnels de celle-ci.

Selon encore un autre objet, la présente invention se rapporte à l'utilisation d'au moins un peptide conforme à l'invention et/ou un acide nucléique codant pour ladite séquence pour la préparation d'une composition pharmaceutique destinée à la prévention et/ou au traitement d'une infection par un virus de l'immunodéficience humaine chez des patients de phénotype HLA-DR1.

Selon encore un autre objet, la présente invention se rapporte à un peptide selon l'invention associé à un complexe biotinylé composé d'une chaîne α (DRα) et d'une chaine β (DRβ) d'un HLA de type II, d'un bras espaceur et de stréptavidine pour former un tétramère de classe II.

Selon encore un autre de ses aspects, la présente invention se rapporte à une composition pharmaceutique contenant au moins une quantité efficace d'au moins un peptide selon l'invention, en combinaison avec un véhicule, un excipient, un diluant et/ou un adjuvant pharmaceutiquement acceptables.

### Peptides

Les peptides selon l'invention sont des peptides isolés comprenant au moins un épitope consistant en une séquence d'acides aminés choisie parmi SEQ ID NO 1, SEQ ID NO 2, SEQ ID NO 3, et des analogues fonctionnels de celle-ci.

Au sens de la présente invention, on entend par "peptide" une chaîne d'acides aminés reliés au moyen de liaisons peptidiques et pouvant comprendre des acides aminés de type D ou L, et également tout dérivé d'acides aminés, tels que la citrulline, l'ornithine, ou l'acide β-aminobutyrique, ainsi que des acides aminés non naturels qui conviendraient à l'invention. Habituellement, un peptide peut comprendre de 2 à 30 acides aminés, en particulier de 6 à 25, et notamment de 12 à 20 acides aminés.

Au sens de la présente invention, on entend par "épitope", une région d'un antigène reconnu par un anticorps ou un récepteur pour antigènes. Les épitopes sont également dénommés "déterminant antigénique". Un épitope de cellule T est un court peptide (de 6 à 10 acides aminés pour les cellules T CD8+, et de 12 à 24 acides aminés pour les cellules T CD4+) dérivé d'un antigène protéique.

En particulier, les peptides conformes à l'invention sont des peptides isolés comprenant un épitope consistant en une séquence d'acides aminés choisie parmi SEQ ID NO 1, SEQ ID NO 2, SEQ ID NO 3, et des analogues fonctionnels de celle-ci, et présentant une longueur inférieure ou égale à 24 acides aminés.

Plus particulièrement, les peptides selon l'invention sont des peptides isolés consistant en une séquence d'acides aminés choisie parmi SEQ ID NO 1, SEQ ID NO 2, SEQ ID NO 3, et des analogues fonctionnels de celle-ci.

Les analogues fonctionnels, ou équivalents fonctionnels biologiques, sont compris, par l'homme de l'art, comme visant des peptides comprenant des modifications structurelles par rapport aux peptides selon l'invention mais conservant en revanche les caractéristiques immunogéniques (ou propriétés biologiques fonctionnelles) des peptides selon l'invention, qui sont définies par la capacité d'interaction de ces peptides avec les molécules HLA-DR1 et l'induction d'une réponse biologique chez les lymphocytes T CD4+.

Les différentes modifications envisageables peuvent être introduites par des moyens physiques, chimiques et/ou biologiques comme, par exemple, la substitution d'un ou plusieurs acides aminés par d'autres, l'insertion d'un ou plusieurs acides aminés et/ou la délétion d'un ou plusieurs acides aminés.

Selon une variante avantageuse, les analogues fonctionnels des peptides selon l'invention sont sélectionnés parmi ceux présentant des propriétés biologiques fonctionnelles améliorées.

En raison de leur taille relativement faible, les peptides conformes à l'invention peuvent être obtenus par n'importe quelle technique de synthèse peptidique connue de l'homme de l'art, telle que par exemple les méthodes de synthèse en solution ou les méthodes de synthèse en phase solide.

En dehors de la synthèse peptidique, il peut également être envisageable d'obtenir les peptides conformes à l'invention par d'autres méthodes, notamment des techniques d'ADN recombinant telles que décrites par la suite.

Selon un autre mode de réalisation, les peptides de la présente invention, ou des analogues de ceux-ci peuvent également être associés à d'autres peptides afin d'en augmenter les propriétés immunogéniques. Par exemple, les peptides conformes à l'invention peuvent être associés, notamment au moyen d'un bras espaceur, à d'autres peptides comprenant des épitopes ayant, par exemple, une activité stimulante au regard de la réponse des lymphocytes T cytotoxiques. A titre d'exemple de peptide comprenant des épitopes dérivés d'antigènes du VIH susceptibles d'activer une réponse à lymphocytes T cytotoxiques, on peut mentionner les peptides cités dans la demande WO 00/29008.

Le bras espaceur peut comprendre des molécules de relativement petites tailles, telles que des acides aminés ou analogues, et qui sont substantiellement neutres dans des conditions physiologiques.

Lorsqu'il est présent, le bras espaceur peut comprendre au moins un ou deux acides aminés, identiques ou différents, et notamment de trois à quinze acides aminés, et en particulier de six à dix acides aminés.

Selon une autre variante, le peptide conforme à l'invention peut être relié à un autre peptide sans bras espaceur.

Selon un autre mode de réalisation de l'invention, au moins un des peptides conformes à l'invention peut être compris dans une construction peptidique multi-épitope.

Un polypeptide ou peptide ou protéine poly-épitope ou multi-épitope peut comprendre, par exemple de 2 à 50 peptides immunogéniques combinés en un polypeptide unique au moyen de techniques recombinantes ou synthétiques, notamment de 4 à 20, et plus particulièrement de 8 à 12 peptides immunogéniques.

Les peptides multi-épitopes peuvent être linéaires ou ramifiés. Ils peuvent être produits au moyen de méthodes de synthèse chimique ou au moyen de technique d'ADN recombinant.

La construction multi-épitopique peut être de type hétéro-polymère ou homo-polymère.

Elle peut comprendre des épitopes stimulant l'activité des lymphocytes T-auxiliaires et/ou des lymphocytes T cytotoxiques.

Les peptides conformes à l'invention sont avantageusement utilisés tels quels ou formulés dans une composition pharmaceutique comme par exemple définie par la suite pour la prévention et/ou le traitement d'une infection à VIH, et en particulier à VIH-1.

### Acides nucléiques et vecteurs d'expression

L'homme de l'art, pour obtenir des peptides conformes à l'invention selon des procédés à base d'ADN recombinant, peut, par exemple, utiliser le manuel "Molecular Cloning - A Laboratory Manual" (2ème édition), Sambrook et al., 1989, Vol. I-III, Coldspring Harbor Laboratory, Coldspring Harbor Press, NY, (Sambrook).

En raison de la dégénérescence du code génétique, il est apprécié que de nombreuses variations conservatrices de constructions d'acides nucléiques permettent d'obtenir des séquences d'acides aminés fonctionnellement identiques.

La conséquence de l'introduction d'une modification dans une séquence d'acides nucléiques conforme à l'invention peut être évaluée notamment par la détermination des propriétés immunogéniques des peptides codées par les séquences d'acides nucléiques au moyen de tout essai immunologique connu, et comprenant éventuellement la comparaison des résultats, obtenus avec des peptides modifiés, avec ceux obtenus avec les peptides non modifiés.

L'obtention des constructions multi-épitopiques précédemment évoquées peut se faire par la ligature de séquences d'acides nucléiques recombinantes, ou synthétiques, codant pour chacun des peptides comprenant un épitope soit par des enzymes (par exemple de type ligase) soit par synthèse chimique.

Ainsi, la présente invention se rapporte aux séquences d'acides nucléiques codant pour au moins un peptide conforme à l'invention, ainsi que pour leurs analogues fonctionnels.

Selon encore un mode de réalisation, au moins une des séquences d'acides nucléiques conformes à l'invention peut être introduite dans tout vecteur d'expression approprié.

Ces vecteurs peuvent être utilisés, par la suite, pour transformer des cellules hôtes et produire les peptides recombinants voulus.

De manière à permettre l'expression d'au moins un peptide recombinant dans les cellules hôtes, la séquence d'acides nucléiques codant pour celui-ci peut être liée, dans un vecteur d'expression, de manière fonctionnelle, respectivement au niveau de ses codons départ et stop, avec une région promotrice et une région terminatrice, et habituellement un système de réplication.

L'utilisation de cellules hôtes, telles que les bactéries, les levures, les cellules d'insecte, de mammifère, ou de plante pour la production des peptides conformes à l'invention en utilisant les vecteurs d'expression précédemment définis, peut également être envisagée dans le cadre de la présente invention.

Avantageusement, les cellules de mammifère dans lesquelles il est possible de faire s'exprimer un vecteur d'expression comprenant un acide nucléique conforme à l'invention, peuvent être par exemple, des cellules présentatrices d'antigènes.

Les vecteurs d'expression contenant les séquences d'acides nucléiques codant pour les peptides conformes à la présente invention peuvent être utilisés pour transformer des cellules hôtes par toute technique appropriée, telle que par exemple, la transfection au phosphate de calcium, ou l'électroporation.

### Anticorps

Selon un autre mode de réalisation, la présente invention se rapporte également à au moins un anticorps susceptible d'être obtenu au moyen d'au moins un peptide conforme à la présente invention. Cet anticorps présente la capacité de se lier spécifiquement à un épitope consistant en une séquence d'acides aminés choisie parmi SEQ ID NO 1, SEQ ID NO 2, SEQ ID NO 3.

Ainsi, les peptides conformes à l'invention peuvent être utilisés pour produire des anticorps au moyen de différentes techniques connues de l'homme de l'art (telles que celles décrites dans Current Protocols in Immunology, Willey/Greene NY ; et Antibodies: Laboratory Manual Harlow, Harlow and Lane, Cold Spring Harbor Laboratory Press, 1989).

Les anticorps ainsi obtenus peuvent être utilisés par exemple à titre d'outils diagnostiques, et/ou thérapeutiques chez des individus susceptibles d'être atteints par une infection à VIH.

Les peptides conformes à l'invention peuvent être utilisés tels quels pour l'obtention d'anticorps monoclonaux ou polyclonaux, ou ils peuvent être associés à d'autres agents immunogènes, par exemple avec d'autres fragments de protéines bactériennes ayant des propriétés immunogéniques, telles que l'hémagglutinine A du virus de l'influenza, ou avec d'autres épitopes dans des constructions peptidiques multi-épitopes ou en association avec une molécule HLA-DR1 ou un fragment de celle-ci.

Selon un mode de réalisation, un anticorps conforme à l'invention peut être de type complexe spécifique, et se lier spécifiquement à un complexe comprenant un épitope conforme à l'invention et une molécule HLA-DR1.

Les anticorps selon l'invention comprennent les fragments d'anticorps ayant la capacité de se lier aux peptides conformes à l'invention (par exemple les fragments Fab), ainsi que des anticorps de type chaîne unique recombinante (par exemple des anticorps scFv).

Les anticorps monoclonaux peuvent être obtenus à partir de cellules sécrétant l'anticorps souhaité, telles que des lymphocytes B en culture, ou des hybridomes.

### Tétramères

Selon encore un autre mode de réalisation, les peptides conformes à l'invention peuvent être utilisés pour préparer des tétramères.

La préparation et l'utilisation de tétramères sont bien connues de l'homme de l'art (voir notamment Novak et al., J. Clin. Invest., 1999, 104:R63-R67 ; Cochran et al., Immunity, 2000, 12:241-250).

Un tétramère comprenant un peptide conforme à l'invention peut être obtenu de la manière suivante : des chaînes lourdes a et β correspondantes de la molécule HLA-DR1, sont repliées en présence de peptides capables de s'y lier pour générer des complexes.

Selon un autre mode de réalisation, il est possible d'utiliser un peptide lié par génie génétique à l'extrémité N-terminale de la chaîne DRβ comme détaillé par Iyasere et al., (J. Virol., 2003).

Le complexe est ensuite biotinylé à l'extrémité C-terminale de la chaîne lourde DRβ de la molécule HLA-DR1, comportant un site de biotinylation ajouté, par exemple, par des techniques d'ADN recombinant. La formation du tétramère est ensuite induite par addition de stréptavidine.

Les deux chaînes lourdes DRα et DRβ de la molécule HLA-DR1 peuvent être produites soit en cellules d'insectes, par exemple des cellules de drosophile, et reliées, par exemple, par un domaine Leu Zipper, et par interaction biotine-streptavidine-PE (phycoerythrine) (Novak et al., J. Clin. Invest., 1999) soit produites dans des bactéries, par exemple *Escherichia coli* (Cochran JR et al., Immunity, 2000, 12:241-250).

Lorsque de la stréptavidine marquée au moyen d'une sonde fluorescente est utilisée, le complexe tétramérique peut être utilisé pour identifier des cellules spécifiques d'un antigène, par exemple, au moyen de cytométrie en flux.

De tels procédés peuvent être utilisés à des fins de diagnostic et/ou de pronostic de l'état immunitaire d'un individu de phénotype HLA-DR1, avant, pendant et/ou après un traitement thérapeutique et/ou prophylactique.

Les cellules identifiées au moyen des procédés précédemment décrits peuvent être utilisées à des fins thérapeutiques, notamment après amplification *in vitro,* et injection chez un individu.

### Cellules

Selon un mode de réalisation, des clones de lymphocytes T-auxiliaires (ou CD4+) isolés et activés au moyen des peptides conformes à la présente invention ou des analogues fonctionnels de ceux-ci, peuvent être utilisés à titre d'agent prophylactique et/ou thérapeutique au regard d'une infection à VIH, et en particulier d'une infection à VIH-1.

Ainsi, selon un de ses objets, la présente invention concerne également, au moins un clone de lymphocyte T-auxiliaire isolé, comprenant un récepteur reconnaissant spécifiquement un complexe contenant une molécule HLA-DR1 et un peptide conforme à l'invention.

La présente invention concerne encore une méthode de traitement et/ou de prévention d'une infection par un virus de l'immunodéficience humaine comprenant au moins une étape consistant à administrer à un individu une quantité efficace d'au moins un clone de lymphocyte T-auxiliaire conforme à l'invention.

Au sens de la présente invention, on entend par "clone" ou "population clonale" un groupe de cellules génétiquement identiques, dérivées d'une cellule unique. Le récepteur des cellules T (ou TcR) présent à la surface de chacune des cellules T de la population clonale est identique et reconnaît de manière spécifique une portion précise d'un antigène donné.

Les lymphocytes T-auxiliaires peuvent être obtenus à partir de cellules mononuclées du sang périphérique (PBMC) prélevées au moyen d'une cytaphérèse.

Les lymphocytes T-auxiliaires activés peuvent être obtenus par culture de cellules précurseurs en présence d'une source de cellules présentatrices d'antigène, telles que des cellules dendritiques, et des peptides immunogéniques appropriés. Les cellules présentatrices d'antigène peuvent être, avantageusement, rendues incapables de se multiplier après irradiation (par exemple par exposition à environ 5000 rads). Le cas échéant, des cellules nutritives (telles que des cellules B autologues irradiées) peuvent également être présentes. Après une période d'incubation appropriée (par exemple, environ de 7 à 28 jours), au cours de laquelle les cellules précurseurs sont activées et prolifèrent sous la forme de cellules effectrices, les cellules obtenues, à savoir les lymphocytes T-auxiliaires activés, peuvent être reperfusées chez l'individu à partir duquel elles ont été prélevées, afin d'induire une réponse immunitaire à l'encontre du virus de l'immunodéficience humaine.

Selon une autre variante, les cellules ainsi obtenues peuvent être reperfusées chez un individu différent de celui ayant fourni les cellules, mais étant compatible ou rendu compatible au niveau du HLA

Les cellules présentatrices d'antigène peuvent être des cellules dendritiques, des lymphocytes B, ou toutes autres cellules portant à la surface des molécules HLA-DR1.

A titre d'exemple de procédé permettant d'obtenir des lymphocytes convenant à la mise en oeuvre de l'invention, il est possible de mentionner le procédé décrit dans la demande de brevet WO 01/29190.

Par ailleurs, les cellules présentatrices d'antigène peuvent être également utilisées en tant que moyen permettant d'obtenir une réponse immunitaire. Ces cellules peuvent être chargées au moyen des peptides conformes à l'invention, et/ou de leurs analogues fonctionnels, et/ou des constructions multi-épitopiques, et/ou des séquences d'acides nucléiques conformes à l'invention.

Ainsi, la présente invention a encore pour objet au moins une cellule présentatrice d'antigène isolée, portant à la surface des molécules HLA-DR1, associée à au moins un peptide et/ou au moins un acide nucléique conforme à l'invention.

Les cellules présentatrices d'antigène convenant à la mise en oeuvre de l'invention, peuvent être par exemple les cellules dendritiques, les lymphocytes B, les macrophages, ainsi que les cellules présentatrices d'antigène dites artificielles.

A titre d'exemple de procédés permettant d'obtenir des cellules présentatrices d'antigène, il est possible de mentionner les techniques décrites dans les demandes de brevet WO 97/44441, WO 03/089629, WO 93/20185 et WO 97/29182 ou décrites par Brossart et al., (Blood, 1998, 92:4238-47).

### Compositions pharmaceutiques

Les peptides conformes à la présente invention et les analogues fonctionnels de ceux-ci, peuvent également être utilisés pour la préparation de compositions pharmaceutiques destinées à la prévention et/ou au traitement d'une infection par virus de l'immunodéficience humaine chez des patients HLA-DR1. En particulier le virus de l'immunodéficience humaine peut-être VIH-1.

Selon un mode particulier de réalisation, la composition pharmaceutique est avantageusement un vaccin.

Les compositions pharmaceutiques conformes à la présente invention peuvent comprendre, en combinaison avec un véhicule, un excipient, un diluant et/ou un adjuvant pharmaceutiquement acceptables, un peptide conforme à l'invention, un analogue fonctionnel de celui-ci, un peptide multi-épitope tel que défini précédemment, une séquence d'acides nucléiques telle que définie précédemment, le cas échéant dans un vecteur d'expression, un anticorps tel que défini précédemment, et leurs mélanges. Ces éléments seront par la suite désignés par l'expression " agent(s) actif(s)".

Les excipients susceptibles de convenir à la mise en oeuvre d'une composition pharmaceutique conforme à l'invention peuvent être, à titre d'exemple, des agents tamponnants et d'ajustement du pH, des agents d'ajustement de la tonicité du milieu, des agents mouillants.

Les compositions pharmaceutiques conformes à la présente invention peuvent être administrées par voie locale, par voie orale, par voie topique ou par voie parentérale, telle que par exemple, la voie intraveineuse, sous-cutanée, intradermique ou intramusculaire.

Ainsi, les compositions pharmaceutiques conformes à l'invention peuvent comprendre, un agent actif tel que défini précédemment, avantageusement dissout ou suspendu dans un véhicule stérile pharmaceutiquement acceptable, tel que par exemple un milieu aqueux.

La solution aqueuse ainsi obtenue peut être ensuite conditionnée pour un usage tel quel ou lyophilisé. La préparation lyophilisée est ensuite combinée avec une solution aqueuse stérile, avant son administration.

Dans le cadre d'une application thérapeutique, les compositions conformes à l'invention peuvent être administrées à un individu déjà infecté avec le VIH en une quantité suffisante pour stopper la propagation du virus dans l'organisme ou, au moins, partiellement, stopper les symptômes de la maladie et ses complications.

Au regard du SIDA, le terme "traitement" se rapporte à une diminution de la sévérité de la maladie, par exemple, en réduisant la disparition des lymphocytes T CD4+.

Au regard du SIDA, le terme "prévenir" se rapporte au fait de prévenir l'apparition du SIDA, par exemple en administrant une composition pharmaceutique conforme à l'invention, avant le développement de la maladie. Cela indique que les compositions pharmaceutiques conformes à la présente invention peuvent être utilisées à titre d'agent prophylactique pour empêcher, notamment, la réduction du nombre de lymphocytes T CD4+.

Une quantité adéquate de l'un des agents actifs permettant d'accomplir cela est définie comme étant une quantité efficace.

La quantité efficace d'agent actif est à ajuster selon la nature dudit agent, la sévérité de la maladie, la composition à injecter, le poids et l'état général du patient.

A titre d'exemple, pour un patient d'environ 70 kg, la quantité efficace, par exemple dans le cadre d'un peptide selon l'invention, pourra varier d'environ 100 µg/infection/jour à environ 3000 µg/injection par jour, et être notamment d'environ 1500 µg/injection par jour en une ou plusieurs dose(s).

Selon un mode de réalisation, le mode d'administration peut comprendre une primoinjection, suivie d'un ou plusieurs rappels.

Selon une autre variante de réalisation, les agents actifs tels que définis précédemment peuvent être associés à des vecteurs d'administration (ou véhicules) tels que des liposomes, des exosomes, ou des cellules présentatrices d'antigène, telles que définies précédemment (comme les cellules dendritiques ou les macrophages), ou d'un mélange de ceux-ci. Les agents actifs associés aux vecteurs peuvent être disposés à l'intérieur et/ou à la surface de ceux-ci.

L'administration au moyen de liposomes peut permettre de cibler les agents actifs vers des tissus particuliers, tels que les tissus lymphoïdes. Les liposomes peuvent permettre également, avantageusement, d'augmenter la demi-vie des agents actifs.

Dans les compositions pharmaceutiques comprenant à titre de vecteur d'administration des liposomes, les agents actifs peuvent être incorporés dans les liposomes, seuls ou en combinaison avec une molécule favorisant la liaison avec par exemple des cellules lymphoïdes et/ou myéloïdes, telle que des anticorps monoclonaux se liant à des marqueurs de surface, tels que par exemple, CD45, CD83 ou CD86, ou DC-SIGN ou L-SIGN. Ainsi, les liposomes associés à des agents actifs selon l'invention, peuvent être dirigés vers les tissus comprenant des cellules lymphoïdes et/ou myéloïdes et ainsi libérer l'agent actif de manière plus efficace.

De nombreuses méthodes sont connues de l'homme de l'art pour préparer des liposomes, telles que par exemple les méthodes décrites dans US 4,235,971 ou US 4,837,028.

Une suspension de liposome peut être administrée par voie intraveineuse, localement, ou de manière topique selon une quantité qui dépend, notamment, de l'agent actif à délivrer, et de l'état de l'individu à traiter.

Les agents actifs peuvent être également administrés sous la forme d'exosomes. Les exosomes sont des vésicules d'environ 50 à 90 nm obtenues à partir de cellules dendritiques lors de leur maturation. Ces vésicules possèdent une composition en protéines caractéristique, comprenant notamment des molécules HLA de type I et II, ainsi que d'autres molécules de co-stimulation, permettant ainsi l'activation de la réponse immunitaire innée et adaptative.

Les exosomes peuvent être obtenus à partir de cellules dendritiques dérivées de cellules mononuclées du sang périphérique, prélevées, par exemple, par cytaphérèse, et par la suite cultivées en milieu comprenant de l'IL4 et du GM-CSF, ou selon les conditions définies dans la demande WO 97/44441.

Les exosomes convenant à la mise en oeuvre de la présente invention peuvent être préparés, par exemple, selon le procédé décrit dans le brevet US 6,812,023.

La présente invention a également pour objet l'utilisation d'au moins un agent actif tel que défini précédemment, tel qu'un peptide et/ou un acide nucléique conforme à l'invention, pour la préparation d'une composition pharmaceutique destiné à la prévention et/ou au traitement d'une infection par virus de l'immunodéficience humaine, et en particulier le VIH-1, chez des patients de phénotype HLA-DR1.

La présente invention se rapporte également à une méthode de traitement et/ou de prévention d'une infection par un virus de l'immunodéficience humaine comprenant au moins une étape consistant à administrer à un individu une quantité efficace d'au moins un agent actif tel que défini précédemment comme un peptide et/ou un acide nucléique conforme à l'invention.

Le peptide et/ou l'acide nucléique conforme à l'invention mise en oeuvre dans cette méthode peut être compris dans une composition pharmaceutique telle que définie précédemment.

### Méthode de diagnostic

Selon un autre mode de réalisation, la présente invention concerne également une méthode de diagnostic pour déterminer l'état immunitaire d'un individu susceptible de présenter une infection par un virus de l'immunodéficience humaine, en particulier le VIH-1, comprenant au moins les étapes consistant à :
- mettre en contact *in vitro* des lymphocytes T-auxiliaires isolés dudit individu avec un peptide conforme à la présente invention ou un analogue fonctionnel de celui-ci, et
- évaluer une réponse biologique.

La mise en contact, *in vitro,* de lymphocytes T-auxiliaires isolés avec un peptide conforme à l'invention, ou des analogues fonctionnels de celui-ci, peut se faire directement ou au moyen de cellules présentatrices d'antigène, telle que définies précédemment.

Selon un mode de réalisation, l'individu est de phénotype HLA-DR1.

La réponse biologique évaluée peut être, par exemple, un indice de prolifération (ou de stimulation), la sécrétion d'une ou plusieurs molécules biologiques dans le milieu extracellulaire ou dans le cytoplasme, l'apparition et/ou la disparition d'une ou plusieurs molécules associées aux cellules, et une combinaison de ces réponses.

A titre d'exemple, les réponses biologiques obtenues chez les lymphocytes T-auxiliaires peuvent être évaluées selon les procédés décrits dans la partie expérimentale du présent texte ou dans la demande de brevet WO 04/050909.

Les molécules biologiques associées aux lymphocytes T-auxiliaires susceptibles d'être détectées peuvent être par exemple des cytokines et/ou des chémokines et/ou des enzymes et/ou des déterminants de surface permettant de caractériser un phénotype d'une population de cellules T-auxiliaires particulières. Parmi les cytokines, on peut citer à titre d'exemple l'IFN-γγ, l'IL-2, l'IL-4, l'IL-5, l'IL-10.

Les molécules biologiques sécrétées par les lymphocytes T-auxiliaires, suite à leur activation, peuvent être également détectées au moyen de la technique ELISPOT.

Par exemple, l'IFN-γγ peut être détecté au moyen d'un test ELISPOT, après incubation des cellules T (en présence ou non d'autres cellules mononuclées du sang périphérique) avec des peptides conformes à l'invention, comme décrit dans Forsthubert et al., (Science, 1996, 271:1728-30).

A titre d'exemple de déterminants de surface dont l'apparition ou la disparition (ou la présence ou l'absence) peut être mesurée, il peut être fait mention de CD4, CD28, CD69, CTLA-4, CD45-RA, CD45-RO, CD62-L

La détection des déterminants de surface peut se faire au moyen de toute technique connue de l'homme de l'art, notamment au moyen de cytométrie en flux, après marquage des molécules à détecter au moyen de sondes fluorescentes, par exemple d'anticorps fluorescents.

A titre de réponse biologique, il est également possible de mesurer l'indice de prolifération (ou de stimulation) des lymphocytes T-auxiliaires après mise en contact, in *vitro,* avec un ou des peptide(s) conforme(s) à l'invention ou des analogues fonctionnels de ceux-ci.

La mesure de la prolifération (ou de stimulation) peut se faire, par exemple, par mesure de l'incorporation de la [³H]-thymidine (comme décrit dans la partie expérimentale du présent texte) ou par la méthode de la dilution d'une sonde fluorescente, telle que décrite dans la demande WO 04/050909, ou par Lyons, (J. Immunol. Methods, 2000, 243:147-154) ou Givan et al., (J. Immunol. Methods, 1999, 230:99-112).

La présente invention se rapporte également à une méthode de diagnostic permettant de déterminer l'état immunitaire d'un sujet susceptible de présenter une infection par un virus de l'immunodéficience humaine, comprenant au moins l'étape consistant à quantifier, *in vitro,* dans une population de lymphocytes isolés dudit sujet, des lymphocytes susceptibles d'être activés par mise en contact avec un complexe contenant une molécule HLA-DR1 et un peptide conforme à l'invention, ou des analogues fonctionnels de celui-ci.

Cette quantification peut se faire avec ou sans étape préalable de stimulation des cellules lymphocytes isolés du sujet.

Ainsi, après prélèvement des lymphocytes T chez un sujet, la détection peut ensuite s'opérer, par exemple, par cytométrie en flux, à l'aide d'anticorps marqués par une sonde fluorescente et spécifiques de ce récepteur.

Selon une autre variante, il est possible de détecter ces lymphocytes T-auxiliaires au moyen d'un tétramère, par exemple tel que décrit précédemment, marqué par une sonde fluorescente, par exemple, selon le protocole décrit, par Novak et al., J. Clin. Invest., 1999, 104:R63-R67 ou par Cochran et al., Immunity, 2000, 12:241-250.

La présente invention se rapporte également à un kit de diagnostic permettant, notamment, de mettre en oeuvre les méthodes précédemment décrites et comprenant au moins un peptide conforme à la présente invention, ou des analogues fonctionnels de celui-ci.

La présente invention se rapporte également à un kit de diagnostic permettant, notamment, la mise en oeuvre des méthodes décrites précédemment et comprenant au moins un tétramère tel que défini précédemment.

L'invention décrite ici est susceptible de variations et de modifications. L'invention inclut ces variations et modifications.

L'invention pourra être mieux comprise à la lecture des exemples suivants. Ceux-ci, cependant, ne doivent pas être interprétés comme limitant la portée de l'invention.

### Légendes des figures

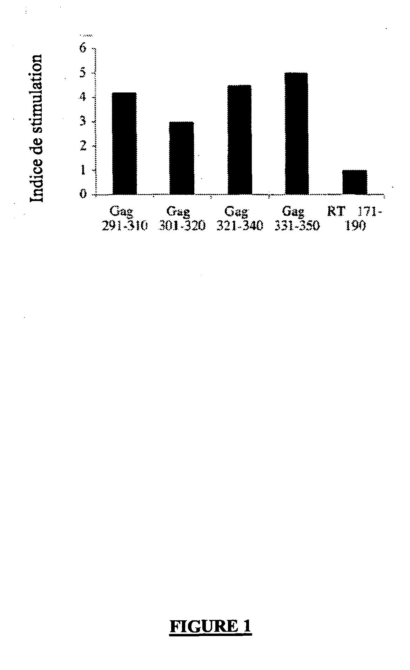

Figure 1 : Est représenté sur cette figure l'Indice de Stimulation de lymphocytes T CD4+ issus de souris transgéniques HLA-DR1, invalidées H-2 - classe II (IAβ^{bo}) et immunisées au moyen de la protéine p24 du HIV. Les cellules T CD4+ ont été restimulées *in vitro* par des blastes activés par du LPS et chargés en peptide Gag₂₉₁₋₃₁₀, Gag₃₀₁₋₃₂₀, Gag₃₂₁₋₃₄₀, Gag₃₃₁₋₃₅₀ et RT₁₇₁₋₁₉₀ en tant que peptide contrôle. L'Indice de Stimulation représente le rapport de la radioactivité mesuré après incorporation de [³H]-thymidine par les lymphocytes T, en présence de peptides spécifiques, sur la radioactivité mesurée par incorporation de la [³H]-thymidine par les lymphocytes T, en présence du peptide contrôle.

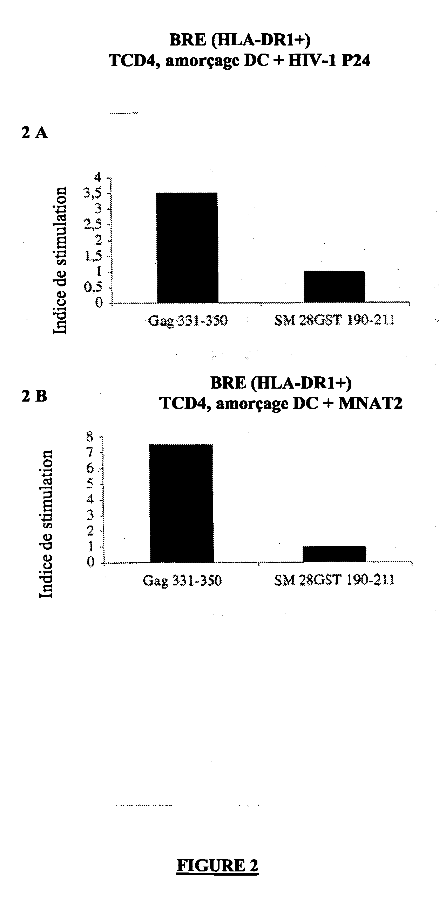

Figure 2 : Sont représentés sur cette figure les Indices de Stimulation de lymphocytes CD4+ isolés de patients HLA-DR1 séronégatifs et amorcés par des cellules dendritiques chargées avec l'antigène p24 du VIH-1 (Figure 2A) ou du VIH-1 MN inactivé par l'aldrithiol-2 (MNAT2) (Figure 2B). Les cellules ont été ensuite stimulées par le peptide Gag₃₃₁₋₃₅₀ ou le peptide contrôle SM 28GST₁₉₀₋₂₁₁.

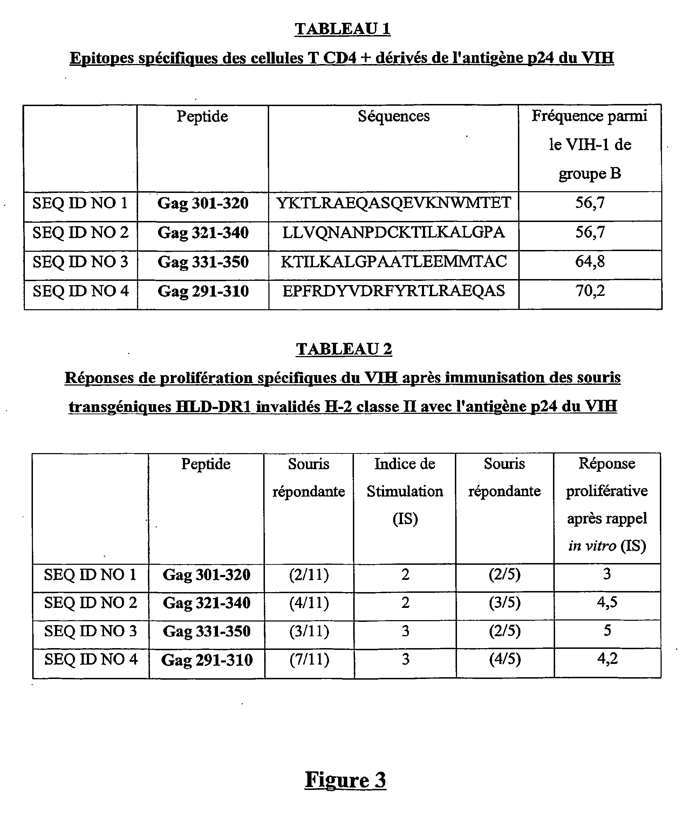

Figure 3 : Tableau 1 : Sont représentées dans ce tableau les séquences des épitopes Gag₃₀₁₋₃₂₀, Gag₃₂₁₋₃₄₀, Gag₃₃₁₋₃₅₀, et Gag₂₉₁₋₃₁₀, ainsi que leur fréquence dans les virus VIH-1 du groupe B.
Tableau 2 : Sont représentés dans ce tableau le nombre de souris répondant aux épitopes Gag₃₀₁₋₃₂₀, Gag₃₂₁₋₃₄₀, Gag₃₃₁₋₃₅₀, et Gag₂₉₁₋₃₁₀ ainsi que l'intensité des réponses de prolifération observées.

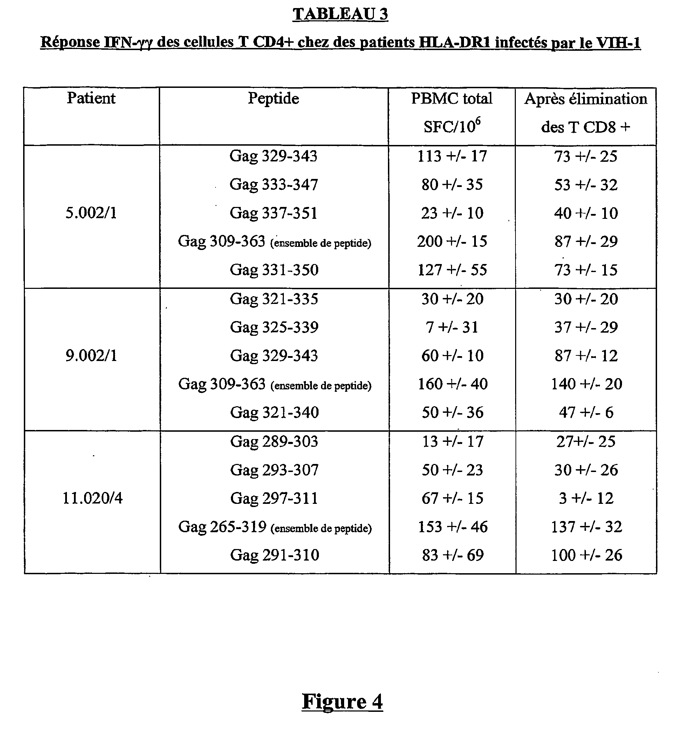

Figure 4: Tableau 3 : Sont représentées dans ce tableau les réponses de sécrétion de l'IFN- γγ par des cellules T CD4+ isolés de patients HLA-DR1 infectés par le VIH-1. Les réponses des cellules T CD4+ humaines spécifiques du VIH-1 ont été évaluées par un test ELISPOT comme décrit dans la section "Matériels et Méthodes". Les réponses ont été évaluées avec et sans élimination des cellules T CD8+. Les peptides utilisés pour stimuler les PBMC étaient soit un ensemble de peptides chevauchant de 15 acides aminés de long (ensemble de 11 peptides consécutifs), soit un peptide de 20 acides aminés de long.

### Matériels et méthodes

### Analyste de séquence

La protéine Gag du VIH-1 a été analysée à l'aide du programme TEPITOPE (Hammer et al., Adv. Immunol., 1997, 66:67-100) pour rechercher la présence de séquences peptidiques de 20 acides aminés de long contenant un motif de liaison au molécule HLA-DR1. Le seuil de prédiction était fixé à 4 %.

### Synthèse de peptide

Des peptides chevauchant de 15 et 20 mers couvrant l'ensemble de la molécule Gag ont été synthétisées et fournies par l'Institut National de la Santé américain (National Institute of Health - NIH USA). Les peptides ont été mis en solution à raison de 1 mg/ml dans un tampon PBS (Phosphate Buffered Saline) contenant 10 % de DMSO.

### Souris transgénique

Des souris transgéniques HLA-DR1, et invalidées H-2 de classe II (IAβ^{bₒ}), dont la lignée a été préalablement établie par Pajot et al., (Int. Immunol., 2004, 16:1275-1282), ont été utilisées lors des différentes expériences.

### Essais d'immunisation et de prolifération

La protéine recombinante p24 du VIH-1 utilisée lors des différentes expériences a été obtenue chez ABCYSS (Paris, France).

Les souris anesthésiées avec 75mg/kg de pentobarbital (Ceva, Santé Animale, Libourne, France) ont reçu 4 µg de protéines recombinantes associées à un adjuvant alun.

Douze jours après la dernière immunisation, les splénocytes, séparés des globules rouges par purification sur Ficoll (5.10⁶ cellules/25 cm² de flacon de culture (TECHNO PLASTIC PRODUCT, TPP, Trasadingen, Suisse)) ont été co-cultivés en présence de cellules blastiques activées par du LPS (5.10⁶ cellules/ flacon de culture), chargées en peptides (20 µg/ml) et irradiées par des rayons γ (180 Gy), en milieu RPMI complémenté avec 10 % de SVF (sérum de veau foetal), HEPES 10 mM, 1mM pyruvate de sodium, 5.10⁻⁵ M mercapto-2éthanol, 100 U.I./ml pénicilline et 100 µg stréptomycine, tels que décrits par Loirat et al, (Journal of Immunology, 2000, 165 :4748-4755).

Les cellules blastiques ont été chargées respectivement avec un des peptides tests Gag₃₀₁₋₃₂₀, Gag₃₂₁₋₃₄₀, ou Gag₃₃₁₋₃₅₀ ou avec un peptide contrôle négatif dérivé d'un antigène du VIH le peptide RT₁₇₁₋₁₉₀, ou avec un petide contrôle positif dérivé de l'antigène p24 du VIH, le peptide Gag₂₉₁₋₃₁₀.

Après sept jours de culture, les essais de prolifération ont été réalisés après distribution de 5.10⁻⁵ cellules/puits en plaques de 96 puits à fond plat des cellules (obtenues chez TTP), en présence de cellules blastiques stimulées par du LPS, irradiées et chargées en peptides (à raison de 2.10⁵ cellules/puits). Les essais de prolifération ont été menés sur une période de 72 heures, dans du milieu RPMI complet complémenté avec 3 % de SVF.

Les cellules ont été incubées, pendant les 16 dernières heures, avec 1 µCi de [³H]-thymidine par puits avant d'être récupérées sur filtres avec un collecteur TOMTEC (Perkin Elmer Applied Biosystem). La radioactivité incorporée a été mesurée à l'aide d'un compteur micro-β (Perkin Elmer Applied Biosystem).

Les résultats sont exprimés en tant qu'Indice de Stimulation (IS) égal au rapport des cpm mesurés après incubation avec les peptides testés sur les cpm mesurés après incubation avec le peptide control.

### Etude sur population

Les individus séronégatifs pour le VIH-1 ont été sélectionnés au regard de leur profil HLA-DR1.

Les individus infectés par le VIH-1 ont été choisis parmi la cohorte française ALT et sélectionnés au regard de leur profil HLA-DR1. La condition de non-progresseur à long terme de ces patients était définie comme étant une infection à VIH asymptomatique pendant au moins 8 ans, avec un comptage de cellules T CD4+ stable à un niveau supérieur ou égal à 600 cellules/mm³, et sans thérapie anti-rétrovirale (Candotti et al., J. Med. Virol., 1999, 58: 256-63).

### Test ELISPOT

La réponse des cellules T CD4+ humaines spécifiques pour le VIH-1 vis-à-vis de différents peptides a été quantifiée par test ELISPOT sur des cellules mononuclées du sang périphérique (PBMC : peripheral blood mononuclear cells) congelées, tel que décrit précédemment (Forsthubert et al., Science, 1996, 271:1728-30).

Des plaques 96 puits ELISPOT (Millipore, Molsheim, France) ont été recouvertes par un anticorps dirigé contre l'IFN-γγ humain (IgG1/B-B1, Diaclone, Strasbourg, France).

Après une étape de blocage en présence d'un milieu contenant 10 % de SVF, 1.10⁵ PBMC ont été distribuées dans les puits. L'expérience à été réalisée en triplicata. Les cellules ont été cultivées la nuit suivant leur décongélation. Puis, les plaques ont été incubées à 35 °C pendant 18 heures en présence de 2 µg/ml de peptides dérivés de la protéine Gag de VIH-1 (peptide chevauchant de 15-mer ou 20-mer ; cf Tableau 3).

La phytohémagglutinine (Murex, Paris, France 1 µg/nl) et le milieu seul ont été respectivement utilisés en tant que contrôle positif et négatif

Les puits ont été par la suite lavés et les spots ont été détectés après addition de l'anticorps de détection dirigé contre l'IFN-γγ humain et marqué à la biotine (B-G1-Diaclone) (4 heures, 37 °C), suivie par l'addition de la phosphatase alcaline fixée sur la stréptavidine (1 heure, 37 °C) et du substrat (5-bromo-4-3-indolyl-phosphate/4-nitroblutétrazolium ; Sigma, Saint-Quentin Falavier, France). L'incubation a été maintenue à température ambiante, jusqu'à l'apparition des points bleus. La fréquence des cellules formant des spots (SFC) spécifiques des antigènes a été mesurée à l'aide d'un système de microscopie automatisée (ZEISS, Munich, Allemagne) et comptée comme positive si un minimum de 50 SFC/1.10⁶ de PBMC étaient détectées au-dessus du bruit de fond. Les tests ELISPOT ont été répétés après élimination des cellules T CD8+ à l'aide de billes magnétiques anti-CD8 (Dynabeads CD8, Dynal). Le fait que plus de 99,8 % des cellules CD8+ étaient éliminées des PBMC, a pu être vérifié par cytométrie en flux (EPICS, XL COULTER).

### Préparation des cellules dendritiques (DC) et chargement en antigènes

Les cellules dendritiques ont été préparées à partir des PBMC telles que décrites précédemment par Brossart et al., (Blood, 1998, 92:4238-47). Les PBMC ont été isolées à partir du sang de donneurs sains HLA-DRB1*01⁺ par centrifugation sur gradient Ficoll-Hypaque.

Les PBMC ont été mises en culture en plaques 6 puits (10-15.10⁶ cellules/puits) et laissées adhérer pendant 1 heure à 37 °C. Les cellules non adhérentes ont été éliminées et les monocytes adhérents ont été cultivés dans du RPMI contenant 10 % de SVF, de la pénicilline/stréptomycine (50 unités/ml - 50 µg/ml), de l'Hepes 10 mM, de la glutamine 10 mM, de l'IL-4 1000 unités/ml (R & D, France) et du GM-CSF 500 unités/ml (Leucomax, Aventis, France). Les DC de la préparation présentaient un phénotype de cellules immatures : CD1a⁺, CD4⁺, CD14⁻, CD83⁺ low, CD86⁺ low, HLA-DR⁺, DC-SIGN⁺. Par la suite les DC ont été congelées (dans du PBS contenant 4 % d'albumine humaine, LFB, France) ou chargées avec des antigènes.

Les DC ont été chargées avec la protéine p24 recombinante de VIH (10 g/ml, Sigma, France) ou du VIH-1 MN inactivé par de l'aldrithiol-2 (AT-2) (1000 ng de p24/ml) avec les agents de maturation Ribomunyl^{®} (1 µg/ml) et de l'IFN-γγ (500 unités/ml) pendant deux heures à 37 °C.

### Amorçage des cellules T

Les cellules T CD4+ ont été isolées à partir de la fraction non adhérente des PBMC, par élimination négative en utilisant les billes magnétiques (Miltenyi Biotec, France). Elles ont été conservées congelées, puis décongelées avant la stimulation des cellules T.

Les cellules T CD4+ purifiées on été incubées en présence de DC autologues matures, irradiées (5000 rad) et chargées en antigènes (dans un rapport de 5/1), ainsi qu'en présence de cellules B autologues irradiées (5000 rad) (dans un rapport de 1/2) dans du milieu RPMI contenant 10 % de sérum humain (Institut Jacques BOY, France) contenant des acides aminés non essentiels (GIBCO, France), du pyruvate de sodium (GIBCO, France) 1mM, 10 mM Hepes, 10 mM glutamine.

Du rhIL-2, à raison de 100 unités/ml (Proleukin, Chiron, France) a été rajouté 5 jours après la stimulation.

Dix jours après la stimulation, les cultures de cellules T ont été restimulées avec des DC irradiées, dans un rapport de 1/20, chargées avec le peptide Gag₃₃₁₋₃₅₀ ou le peptide contrôle SM 28GST₁₉₀₋₂₁₁ (épitope 190 à 211 de l'antigène de Schistosoma mansoni 28 kDa glutathione S-transferase) ainsi qu'en présence de cellules nutritives autologues selon un rapport de 1/5.

Les cultures de cellules T CD4+ ont été par la suite restimulées tous les 10-15 jours avec des cellules B autologues transformées avec EBV (Epstein-Barr Virus, cellules B-EBV) irradiées et chargées en peptides, selon un rapport de 1/2, et de PBMC allogéniques irradiés, selon un rapport de 1/4.

### Test de prolifération des cellules T humaines

Les cellules T en cultures ont été mises en contact avec le peptide Gag₃₃₁₋₃₅₀ ou le peptide SM 28GST₁₉₀₋₂₁₁ à raison de 50 µg/ml pendant 3 heures à 37 °C, puis distribuées dans des plaques 96 puits à fond plat (5-10⁵ cellules/puits). Les cellules ont été cultivées dans du milieu pour cellule T en absence de rhIL-2 pendant 3 jours avant d'être pulsées avec 1 µCi [³H]-thymidine pendant 20 heures.

Les cellules ont été récupérées sur des filtres en utilisant un collecteur TOMTEC (Perkin Elmer Applied Biosystem) et la radioactivité incorporée a été mesurée à l'aide d'un compteur micro-β (Perkin Elmer Applied Biosystem).

Les résultats sont exprimés sous la forme d'un indice de stimulation (SI) représentent le rapport des cpm mesurés après incubation avec le peptide testé sur les cpm mesurés après incubation avec le peptide contrôle.

### Exemple I

### Identification et antigénicité de nouveaux épitopes HLA-DR1 chez des souris transgéniques HLA-DR1

Pour identifier de nouveaux peptides ayant une spécificité HLA-DR1, la séquence d'acides aminés de la protéine Gag a été scannée à la recherche de motifs spécifiques HLA-DR1 à l'aide de l'algorithme TEPITOPE.

Des peptides de 20 acides aminés contenant une région coeur de 9 acides aminés comprenant un motif HLA-DR1 et des acides aminés N et C terminaux supplémentaires ont été choisis, parmi lesquels se trouvent trois nouveaux peptides Gag₃₀₁₋₃₂₀, Gag₃₂₁₋₃₄₀, Gag₃₃₁₋₃₅₀ et un peptide connu Gag₂₉₁₋₃₁₀ (Iyasere et al., J. Virol., 2003, 77 :10900-9) (Tableau 1). Le Tableau 1 indique également la fréquence selon laquelle les séquences identifiées sont présentes dans la souche VIH-1 du groupe B. Cette fréquence, traduisant le pourcentage de conservation, est déterminée sur le nombre de souche VIH présentant la même séquence en acides aminés appartenant au groupe B (HIV databases, Los Alamos National Library and National Institutes of Health, http://hiv-web.lanl.gov/cgi-bin/EPILIGN/epilign.cgi).

Pour évaluer l'immunogénicité des épitopes restreints HLA-DR1, Gag₃₀₁₋₃₂₀, Gag₃₂₁₋₃₄₀ et Gag₃₃₁₋₃₅₀, les souris transgénique HLA-DR1 et invalidées H-2 classe II ont été immunisées avec la protéine p24 du VIH. Par la suite, les cellules T issues de la rate ont été restimulées *in vitro* avec, les peptides à tester ou un peptide contrôle positif Gag₂₉₁₋₃₁₀, ou un peptide contrôle négatif RT₁₇₁₋₁₉₀.

La figure 1 montre que les souris transgéniques HLA-DR1 et invalidées H-2 classe II sont capables de développer une réponse cellulaire proliférative à l'encontre des peptides restreints HLA-DR1 Gag₂₉₁₋₃₁₀, précédemment identifié par Iyasere et al. (J. Virol., 2003, 77:10900-9) comme étant un peptide immunodominant restreint HLA-DR1 capable d'induire une réponse chez des cellules T CD4+ isolées d'individu HLA-DR1 infectés par le VIH, ainsi que contre les peptides Gag₃₀₁₋₃₂₀, Gag₃₂₁₋₃₄₀ et Gag₃₃₁₋₃₅₀.

Aucune réponse n'a pu être induite chez les cellules T CD4+ stimulées au moyen du peptide contrôle dérivé de l'antigène RT du VIH-1.

Le tableau 2 montre que deux souris transgéniques HLA-DR1 et invalidées H-2 classe II sur 11 répondent aux peptides Gag_{301-320,} 4 souris sur 11 répondent aux peptides Gag₃₂₁₋₃₄₀ et 3 souris sur 11 répondent aux peptides Gag₃₃₁₋₃₅₀ avec un indice de stimulation allant de 2 à 3.

De plus un rappel supplémentaire *in vitro* avec ces peptides permet d'augmenter l'indice de prolifération de 3 à 5, confirmant ainsi la spécificité de la réponse.

### Exemple II

### Reconnaissance des épitopes Gag₃₂₁₋₃₄₀ et Gag₃₃₁₋₃₅₀ de p24 VIH-1 par des patients HLA-DR1+ infectés par le VIH

La reconnaissance des épitopes identifiés par des cellules CD4+ isolées de patients HLA-DR1+ infectés par le VIH-1 a été évaluée en utilisant le test ELISPOT. Des PBMC ont été isolées chez un groupe de patients répondant tous à l'antigène entier p24 et/ou à un ensemble de peptides chevauchant de 15 mers couvrant la protéine p24 (Tableau 3).

Ainsi, que le montre le tableau 3, l'épitope Gag₂₉₁₋₃₁₀, précédemment décrit comme étant un épitope HLA-DR1, est capable de stimuler les cellules T CD4+ chez le patient 11.020/4, et d'induire une réponse positive et non altérée après élimination des cellules CD8+.

Le peptide Gag₃₃₁₋₃₅₀ est reconnu par les cellules T CD4+ du patient 5.002/1 autant que l'ensemble de peptides de 15 mers comprenant l'épitope Gag₃₃₁₋₃₅₀.

Le peptide Gag₃₂₁₋₃₄₀ induit une réponse à la limite du seuil de détection par les cellules T CD4+ chez le patient 9.002/1, qui reconnaissent également l'ensemble des peptides avec le même ordre de grandeur.

Ainsi, les deux nouveaux épitopes restreints HLA-DR1, Gag₃₂₁₋₃₄₀ et Gag₃₃₁₋₃₅₀, qui sont immunogéniques chez la souris transgénique HLA-DR1, invalidés H-2 classe II, peuvent également induire une réponse chez des patients HLA-DR1 infectés par le VIH.

### Exemple III

### Stimulation de cellules CD4+ isolées d'un individu HLA-DR1 séronégatif par l'épitope Gag₃₃₁₋₃₅₀

Les PBMC d'un individu HLA-DR1 (individu BRE) séronégatif VIH-1 ont été isolées et amorcées, *in vitro,* avec des DC autologues (chargées avec la protéine p24 du VIH ou le virus entier MN inactivé par l'aldrithiol-2 (MNAT2)) et les cellules T CD4+ ont été séparées des autres cellules et mises en culture.

Les cellules T CD4+ ont été ensuite cultivées *in vitro* pendant 2 semaines en présence de cellules présentatrices d'antigènes, les cellules B-EBV autologues irradiées, et chargées à l'aide du peptide p24 de VIH ou du virus MNAT2.

Puis, les cellules T CD4+ ont été testées au regard d'une réponse proliférative spécifique induite par le peptide Gag₃₃₁₋₃₅₀ ou le peptide contrôle SM 28GST₁₉₀₋₂₁₁.

Après trois jours d'incubation avec l'antigène, l'incorporation de [³H]-thymidine a été mesurée. Un indice de stimulation supérieur à 2 était considéré comme étant significatif

Ainsi que le montre la Figure 2, les cellules T CD4+ de l'individu BRE ont proliféré en réponse au peptide Gag₃₃₁-₃₅₀, mais aucune réponse n'a été observée en présence du peptide contrôle SM 28GST₁₉₀₋₂₁₁ ou du milieu seul. L'indice de stimulation est égal à 10.

Ainsi, l'épitope Gag₃₃₁₋₃₅₀, également immunogénique chez les souris transgéniques HLA-DR1 et invalidé H-2 classe II est capable d'amorcer une réponse de cellules T CD4+ chez un donneur HLA-DR1 séronégatif VIH-1.

## Revendications

1. Peptide comprenant un épitope consistant en une séquence d'acides aminés choisie parmi SEQ ID NO 1, SEQ ID NO 2, SEQ ID NO 3, ledit peptide étant associé à un complexe biotinylé composé d'une chaîne α (DRα) et d'une chaîne β (DRβ) d'un HLA de type II, d'un bras espaceur, et de stréptavidine pour former un tétramère de classe II.

2. Anticorps capable de lier spécifiquement un épitope consistant en une séquence d'acides aminés choisie parmi SEQ ID NO 1, SEQ ID NO 2, SEQ ID NO 3 ou un complexe comprenant ledit épitope et une molécule HLA-DR1.

3. Utilisation d'au moins un peptide comprenant au moins un épitope consistant en une séquence d'acides aminés choisie parmi SEQ ID NO 1, SEQ ID NO 2, SEQ ID NO 3 et/ou un acide nucléique codant pour ladite séquence pour la préparation d'une composition pharmaceutique destinée à la prévention et/ou au traitement d'une infection par un virus de l'immunodéficience humaine chez des patients de phénotype HLA-DR1.

4. Utilisation selon la revendication précédente, dans laquelle le peptide et/ou l'acide nucléique est associé à un véhicule choisi parmi un liposome, un exosome, une cellule présentatrice d'antigène portant à la surface des molécules HLA-DR1.

5. Utilisation selon la revendication précédente, dans laquelle la cellule présentatrice d'antigène est une cellule dendritique ou un macrophage.

6. Cellule présentatrice d'antigène isolée, portant à la surface des molécules HLA-DR1, associées à au moins un peptide comprenant au moins un épitope consistant en une séquence d'acides aminés choisie parmi SEQ ID NO 1, SEQ ID NO 2, SEQ ID NO 3 et/ou un acide nucléique codant pour ladite séquence.

7. Clone de lymphocyte T-auxiliaire isolé, comprenant un récepteur reconnaissant spécifiquement un complexe contenant une molécule HLA-DR1 et un peptide comprenant un épitope consistant en une séquence d'acides aminés choisie parmi SEQ ID NO 1, SEQ ID NO 2, SEQ ID NO 3.

8. Méthode de diagnostic pour déterminer l'état immunitaire d'un individu susceptible de présenter une infection par un virus de l'immunodéficience humaine comprenant au moins des étapes consistant à :
- mettre en contact *in vitro* des lymphocytes T-auxiliaires isolés dudit individu avec au moins un peptide comprenant au moins un épitope consistant en une séquence d'acides aminés choisie parmi SEQ ID NO 1, SEQ ID NO 2, SEQ ID NO 3, et
- évaluer une réponse biologique.

9. Méthode selon la revendication précédente, dans laquelle la réponse biologique est choisie parmi la prolifération, la sécrétion de molécules biologiques, l'apparition et/ou la disparition de molécule associées auxdites cellules, et une combinaison de ces réponses.

10. Méthode de diagnostic pour déterminer l'état immunitaire d'un individu susceptible de présenter une infection par un virus de l'immunodéficience humaine comprenant au moins l'étape consistant à quantifier, *in vitro,* des lymphocytes isolés dudit individu susceptibles d'être activés par mise en contact avec un complexe contenant une molécule HLA-DR1 et un peptide comprenant un épitope consistant en une séquence d'acides aminés choisie parmi SEQ ID NO 1, SEQ ID NO 2, SEQ ID NO 3.

11. Méthode selon la revendication précédente, dans laquelle l'étape de quantification comprend l'utilisation de tétramères tels que définis selon la revendication 1.

12. Kit de diagnostic comprenant au moins un peptide comprenant au moins un épitope consistant en une séquence d'acides aminés choisie parmi SEQ ID NO 1, SEQ ID NO 2, SEQ ID NO 3.

13. Kit de diagnostic comprenant au moins un tétramère tel que défini selon la revendication 1.

## Claims

1. A peptide comprising an epitope consisting in a sequence of amino acids selected from SEQ ID NO 1, SEQ ID NO 2, SEQ ID NO 3, said peptide being associated with a biotinylated complex consisting of an α chain (DRα) and of a β chain (DRβ) of an HLA of type II, of a spacer arm, and of streptavidin in order to form a Class II tetramer.

2. An antibody capable of specifically binding to an epitope consisting in a sequence of amino acids selected from SEQ ID NO 1, SEQ ID NO 2, SEQ ID NO 3 or to a complex comprising said epitope and an HLA-DR1 molecule.

3. The use of at least one peptide comprising at least one epitope consisting in a sequence of amino acids selected from SEQ ID NO 1, SEQ ID NO 2, SEQ ID NO 3, and/or a nucleic acid coding for said sequence in order to prepare a pharmaceutical composition intended for preventing and/or treating an infection by a human immunodeficiency virus in patients of HLA-DR1 phenotype.

4. The use according to the preceding claim, wherein the peptide and/or the nucleic acid is associated with a carrier selected from a liposome, an exosome, and an antigen presenting cell bearing HLA-DR1 molecules at the surface.

5. The use according to the preceding claim, wherein the antigen presenting cell is a dendritic cell or a macrophage.

6. An isolated antigen presenting cell bearing at the surface, HLA-DR1 molecules, associated with at least one peptide comprising at least one epitope consisting in a sequence of amino acids selected from SEQ ID NO 1, SEQ ID NO 2, SEQ ID NO 3 and/or a nucleic acid coding for said sequence.

7. An isolated helper T lymphocyte clone, comprising a receptor which specifically recognizes a complex containing an HLA-DR1 molecule and a peptide comprising an epitope consisting in a sequence of amino acids selected from SEQ ID NO 1, SEQ ID NO 2, SEQ ID NO 3.

8. A diagnostic method for determining the immune condition of an individual likely to have an infection by a human immunodeficiency virus comprising at least steps of:
- putting helper T lymphocytes isolated from said individual in contact *in vitro* with at least one peptide comprising at least one epitope consisting in a sequence of amino acids selected from SEQ ID NO 1, SEQ ID NO 2, SEQ ID NO 3 and
- evaluating a biological response.

9. The method according to the preceding claim, wherein the biological response is selected from proliferation, secretion of biological molecules, appearance and/or disappearance of molecules associated with said cells, and a combination of these responses.

10. A diagnostic method in order to determine the immune condition of an individual likely to have an infection by a human immunodeficiency virus comprising at least the step consisting of quantitating *in vitro*, lymphocytes isolated from said individual, capable of being activated by putting them in contact with a complex containing an HLA-DR1 molecule and a peptide comprising an epitope consisting in a sequence of amino acids selected from SEQ ID NO 1, SEQ ID NO 2, SEQ ID NO 3.

11. The method according to the preceding claim, wherein the quantitation step comprises the use of tetramers as defined according to claim 1.

12. A diagnostic kit comprising at lest one peptide comprising at least one epitope consisting in a sequence of amino acids selected from SEQ ID NO 1, SEQ ID NO 2, SEQ ID NO 3.

13. A diagnostic kit comprising at least one tetramer as defined according to claim 1.

## Patentansprüche

1. Peptid, umfassend ein Epitop bestehend aus einer Aminosäuresequenz ausgewählt unter SEQ ID NO 1, SEQ ID NO 2, SEQ ID NO 3, worin das Peptid mit einem biotinylierten Komplex verbunden ist, der aus einer α-Kette (DRα) und einer β-Kette (DRβ) eines HLA Typ II, einem Abstandshalterarm, und Streptavidin besteht, um ein Klasse II Tetramer zu bilden.

2. Antikörper, der spezifisch an ein Epitop bestehend aus einer Aminosäuresequenz ausgewählt unter SEQ ID NO 1, SEQ ID NO 2, SEQ ID NO 3 oder an einen Komplex binden kann, der das Epitop und ein HLA- DR1 Molekül umfasst.

3. Verwendung von mindestens einem Peptid, das mindestens ein Epitop umfasst, bestehend aus einer Aminosäuresequenz, ausgewählt unter SEQ ID NO 1, SEQ ID NO 2, SEQ ID NO 3, und/oder einer diese Sequenz codierenden Nukleinsäure, um eine pharmazeutische Zusamrnensetzung herzustellen, die dazu vorgesehen ist, eine Infektion durch ein menschliches Immunschwäche-Virus in Patienten mit HLA-DR-1 Phänotyp zu verhindern und/oder zu behandeln.

4. Verwendung nach dem vorstehenden Anspruch, wobei das Peptid und/oder die Nukleinsäure mit einem Träger verbunden ist, ausgewählt unter einem Liposom, einem Exosom und einer Antigen-präsentierenden Zelle, die auf der Oberfläche HLA-DR1 Moleküle trägt.

5. Verwendung nach dem vorstehenden Anspruch, wobei die Antigen präsentierende Zelle eine dendritische Zelle oder ein Makrophage ist.

6. Isolierte Antigen-präsentierende Zelle, die auf der Oberfläche HLA-DR1 Moleküle trägt, die mit mindestens einem Peptid verbunden sind, das mindestens ein Epitop umfasst, bestehend aus einer Aminosäuresequenz ausgewählt unter SEQ ID NO 1, SEQ ID NO 2, SEQ ID 3, und/oder einer diese Sequenz codierenden Nukleinsäure.

7. Isolierter Klon von T_{Helfer}-Lymphozyten, umfassend einen Rezeptor, der spezifisch einen Komplex erkennt, der ein HLA-DR1 Molekül und ein Peptid enthält, das ein Epitop umfasst, bestehend aus einer Aminosäuresequenz ausgewählt unter SEQ ID NO 1, SEQ ID NO 2, SEQ ID NO 3.

8. Diagnostisches Verfahren zum Bestimmen des immunologischen Zustands eines Individuums, das wahrscheinlich eine Infektion mit einem menschlichen Immunschwäche-Virus aufweist, umfassend mindestens die Schritte:
- Inkontaktbringen von aus dem Individuum isolierten T_{Helfer}-Lymphozyten *in vitro* mit mindestens einem Peptid, umfassend mindestens ein Epitop bestehend aus einer Aminosäuresequenz ausgewählt unter SEQ ID NO 1, SEQ ID NO 2, SEQ ID NO 3, und
- Beurteilen einer biologischen Antwort.

9. Verfahren nach dem vorstehenden Anspruch, wobei die biologische Antwort ausgewählt ist unter Proliferation, Sekretion biologischer Moleküle, Auftreten und/oder Verschwinden von mit den Zellen verbundenen Molekülen, und eine Kombination dieser Antworten.

10. Diagnostisches Verfahren zum Bestimmen des immunologischen Zustands eines Individuums, das wahrscheinlich eine Infektion mit einem menschlichen Immunschwäche-Virus aufweist, umfassend zumindest den Schritt bestehend aus Quantifizieren *in vitro* von aus dem Individuum isolierten Lymphozyten, die durch Inkontaktbringen mit einem Komplex bestehend aus einem HLA-DR1 Molekül und einem Peptid, umfassend ein Epitop bestehend aus einer Aminosäuresequenz ausgewählt unter SEQ ID NO 1, SEQ ID NO 2 SEQ ID NO 3, aktiviert werden können.

11. Verfahren nach dem vorstehenden Anspruch, wobei der Quantifizieruagsschntt die Verwendung von wie in Anspruch 1 definierten Tetrameren umfasst.

12. Diagnostisches Kit, umfassend mindestens ein Peptid, umfassend mindestens ein Epitop bestehend aus einer Aminosäuresequenz ausgewählt unter SEQ ID NO 1, SEQ ID NO 2, SEQ ID NO 3.

13. Diagnostisches Kit, umfassend mindestens ein wie in Anspruch 1 definiertes Tetramer.
